(19) Europäisches Patentamt
European Patent Office
Office européen des brevets

(11) EP 1 319 652 B1

(12) **FASCICULE DE BREVET EUROPEEN**

(45) Date de publication et mention de la délivrance du brevet:
**14.12.2005 Bulletin 2005/50**

(51) Int Cl.[7]: **C07C 241/02**

(21) Numéro de dépôt: **02293069.7**

(22) Date de dépôt: **12.12.2002**

(54) **Procédé de décomposition sélective de l'hydrazine dans un mélange hydrazine/hydrazine substituée/eau**

Verfahren für die selektive Zersetzung von Hydrazin in einer Mischung von Hydrazin/substituierte Hydrazin/Wasser

Process for selective decomposition of hydrazine in a mixture of hydrazine/substituted hydrazine/water

(84) Etats contractants désignés:
**AT BE BG CH CY CZ DE DK EE ES FI FR GB GR IE IT LI LU MC NL PT SE SI SK TR**

(30) Priorité: **14.12.2001 FR 0116217**

(43) Date de publication de la demande:
**18.06.2003 Bulletin 2003/25**

(73) Titulaire: **ISOCHEM**
**75004 Paris (FR)**

(72) Inventeurs:
- **Le Gars, Pierre**
  **31400 Toulouse (FR)**
- **Souyri, Denis**
  **31400 Toulouse (FR)**
- **Delalu, Henri**
  **69002 Lyon (FR)**
- **Berthet, Jacques**
  **69003 Lyon (FR)**

(74) Mandataire: **Ahner, Francis et al**
**Cabinet Régimbeau**
**20, rue de Chazelles**
**75847 Paris cedex 17 (FR)**

(56) Documents cités:
**FR-A- 2 383 916** **US-A- 4 122 671**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 319 652 B1

## Description

La présente invention concerne un procédé de décomposition sélective de l'hydrazine dans un mélange hydrazine/hydrazine substituée/eau. Les hydrazines substituées sont des composés utiles en agrochimie, comme par exemple l'hexahydropiridazine, en pharmacie, comme la N-aminopipéridine ou encore pour des produits phytosanitaires, comme la monométhylhydrazine. Dans toutes ces applications, l'hydrazine substituée peut être sous la forme d'une solution aqueuse mais la solution aqueuse doit être -impérativement exempte d'hydrazine.

Une des voies de synthèse les plus connues pour préparer une hydrazine substituée, la monométhylhydrazine, est le procédé Rashig. Cela consiste à faire réagir, dans un premier temps, de l'ammoniac avec de l'hypochlorite de sodium pour former de la chloramine, composé que l'on fait ensuite réagir avec de la méthylamine pour former de la monométhylhydrazine. Le schéma réactionnel du procédé Rashig est le suivant :

$$NH_3 + NaOCl \rightarrow NH_2Cl + NaOH$$

$$NH_2Cl + CH_3NH_2 \rightarrow CH_3NHNH_2 + HCl$$

L'inconvénient d'une telle synthèse est la formation d'hydrazine comme sous-produit. Il est donc nécessaire, ensuite, de séparer l'hydrazine de la monométhylhydrazine. De telles opérations de séparation sont généralement coûteuses.

Une autre solution pour obtenir une solution aqueuse d'hydrazine substituée, exempte d'hydrazine, est de décomposer l'hydrazine dans le mélange hydrazine/hydrazine substituée/eau.

L'homme du métier connaît déjà des procédés de décomposition de l'hydrazine. Ainsi, le brevet US 4 124 538 décrit un catalyseur capable de décomposer l'hydrazine. Ce catalyseur est constitué d'un métal qui est de l'iridium ou un mélange iridium/ruthénium déposé sur de l'alumine. Ce catalyseur peut aussi être utilisé pour décomposer de la monométhylhydrazine. Il n'est donc pas question ici de décomposition sélective. Il en est de même pour le catalyseur décrit dans le brevet US 4 122 671. Ce catalyseur est constitué d'un support poreux et d'un mélange de ruthénium avec un métal choisi parmi l'iridium et le platine. Le support poreux peut par exemple être constitué d'alumine ou de silice. Un tel catalyseur décompose aussi bien l'hydrazine que la monométhylhydrazine.

L'homme du métier ne connaît donc pas, actuellement, de procédé permettant de décomposer sélectivement l'hydrazine dans un mélange hydrazine/hydrazine substituée/eau, c'est-à-dire de procédé dans lequel la vitesse de décomposition de l'hydrazine est nettement supérieure à la vitesse de décomposition de l'hydrazine substituée. La présente invention a pour objet un tel procédé.

L'invention concerne un procédé de décomposition sélective de l'hydrazine dans un mélange hydrazine/hydrazine substituée/eau, caractérisé en ce que l'on introduit dans le mélange un catalyseur choisi dans le groupe constitué par le nickel supporté sur silice/alumine, le mélange nickel-oxyde de nickel supporté sur silice/alumine et le rhodium supporté sur carbone, puis en ce que l'on chauffe le mélange à une température comprise entre 60°C et 120°C.

Ce procédé permet de décomposer sélectivement l'hydrazine par rapport à l'hydrazine substituée. En effet, en introduisant le catalyseur décrit ci-dessus dans un mélange hydrazine/hydrazine substituée/eau, 95 % de l'hydrazine est décomposée contre seulement 10 % d'hydrazine substituée. Le catalyseur contient de 0,5 % à 70 % en poids d'élément métal par rapport au poids total du catalyseur. On appelle élément métal le métal sous la forme d'oxyde et de métal.

De préférence, le catalyseur contient de 45 % à 70 % en poids d'élément métal par rapport au poids total du catalyseur.

Un catalyseur préféré est le mélange nickel-oxyde de nickel supporté sur silice/alumine.

L'hydrazine substituée est une hydrazine de formule (I)

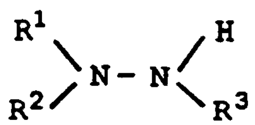

(I)

dans laquelle :

- $R^1$, $R^3$ représentent un atome d'hydrogène ou un groupe alkyl en $C_1$ - $C_6$, linéaire ou ramifié, substitué ou non,
- $R^2$ représente un groupe alkyl en $C_1$ - $C_6$, linéaire ou ramifié, substitué ou non,
- $R^1$ et $R^2$ pouvant former ensemble, avec l'atome d'azote auquel ils sont liés, un hétérocycle, et,
- $R^2$ et $R^3$, lorsque $R^1$ représente un atome d'hydrogène, pouvant former ensemble, avec les deux atomes d'azote, un hétérocycle.

Les substituants de $R^1$, $R^2$ et $R^3$, quand $R^1$, $R^2$ et $R^3$ représentent un groupe alkyle en $C_1$ - $C_6$ sont indépendamment un atome d'halogène, un groupe carboxyle ou un groupe nitrile.

L'hydrazine substituée peut donc être substituée par un groupe alkyle seulement, $R^1$ et $R^3$ représentent alors chacun un atome d'hydrogène et $R^2$ représente un groupe alkyle, linéaire ou ramifié. On pourra citer notamment la monométhylhydrazine, l'isopropylhydrazine et l'isobutylhydrazine.

L'hydrazine substituée pourra également être une hy-

drazine exocyclique, $R^1$ et $R^2$ formant, avec l'atome d'azote auquel ils sont liés, un hétérocycle, c'est le cas pour la N-aminopipéridine.

Enfin, l'hydrazine substituée pourra être une hydrazine endocyclique, $R^2$ et $R^3$ formant ensemble avec les deux atomes d'azote un hétérocycle, $R^1$ représentant un atome d'hydrogène, c'est le cas de l'hexahydropiridazine.

L'hydrazine substituée est de préférence en excès ou en proportion équimolaire par rapport à l'hydrazine, dans le mélange hydrazine/hydrazine substituée/eau. La teneur pondérale en eau est comprise entre 10 % et 90 % en poids par rapport au poids total du mélange.

On donne maintenant un mode de réalisation préférée de l'invention. Le mélange hydrazine/hydrazine substituée/eau est introduit dans un réacteur n'influençant pas la réaction. Le réacteur utilisé est un réacteur en verre ou en PTFE.

Le mélange hydrazine/hydrazine substituée/eau contient de 0,01 à 1 mole d'hydrazine par rapport à l'hydrazine substituée. Les hydrazines substituées préférées sont la monométhylhydrazine, l'isopropylhydrazine, l'isobutylhydrazine, l'hexahydropiridazine et la N-aminopipéridine.

Dans ce même réacteur on introduit le catalyseur choisi dans le groupe constitué par le nickel sur silice/alumine, le mélange nickel/oxyde de nickel sur silice/alumine et le rhodium sur carbone.

Un catalyseur préféré est le mélange nickel-oxyde de nickel supporté par le mélange silice/alumine.

La quantité totale de l'élément métal utilisée pour le catalyseur est comprise entre 0,5 % et 70 % en poids par rapport au poids total du catalyseur, et, de préférence comprise entre 45 % et 70 %.

On chauffe ensuite le milieu réactionnel à une température comprise entre 60°C et 120°C, de préférence entre 80°C et 90°C. On veillera à ce que la pression à l'intérieur du réacteur se situe autour de 1bar. Des pressions supérieures peuvent être utilisées.

Le milieu réactionnel est agité pendant la durée du chauffage.

La durée de chauffage est comprise entre 0,5 heure et 8 heures, elle dépend du catalyseur utilisé. Ainsi, en utilisant le catalyseur nickel-oxyde de nickel supporté sur silice/alumine, il suffit de chauffer 3 heures alors que lorsque l'on utilise le catalyseur rhodium supporté sur carbone il est nécessaire de.chauffer 7 heures.

En fin de chauffage, et quand la température du milieu réactionnel est à température ambiante, on récupère le catalyseur par filtration sous atmosphère inerte, de préférence sous azote.

La solution obtenue est analysée par chromatographie en phase vapeur. Elle contient moins de 0,01 % en poids d'hydrazine par rapport à l'hydrazine substituée.

Les exemples qui suivent illustrent, à titre non limitatif, des possibilités de mise en oeuvre de l'invention.

Exemple 1 : Catalyseur nickel-oxyde de nickel sur silice/alumine

Dans un réacteur de 500ml, on introduit 200ml d'une solution contenant 40 % en poids de monométhylhydrazine, 4 % en poids d'hydrazine et 56 % en poids d'eau.

Dans ce même réacteur on introduit 0,25g de catalyseur nickel-oxyde de nickel supporté par un mélange silice/alumine. Le catalyseur contient 31 % en poids de nickel et 32 % en poids d'oxyde de nickel, soit 56 % en poids d'élément nickel par rapport au poids total du catalyseur.

On chauffe ensuite le milieu à 88°C pendant 3 heures, tout en l'agitant.

En fin de chauffage et lorsque la température du milieu est revenue à température ambiante, on récupère le catalyseur par filtration sous azote et on analyse le milieu par chromatographie en phase gazeuse. On obtient la composition suivante : 39 % en poids de monométhylhydrazine, 0,1 % d'hydrazine et 60,9 % d'eau.

Exemple 2 : Catalyseur rhodium sur carbone

On suit le même mode opératoire que celui décrit dans l'exemple 1, excepté pour la durée de chauffage.

La composition initiale est également identique à celle de l'exemple 1.

Le catalyseur contient 5 % en poids de rhodium par rapport au poids total du catalyseur.

| | |
|---|---|
| Durée de chauffage | 7 heures |
| Composition finale | 38 % monoéthylhydrazine |
| | 0,05 % hydrazine |
| | 61,95 % eau |

Exemples comparatifs 3-5 :

Ces exemples ne font pas partie de l'invention, ils ont été réalisés dans le but de montrer que le fait d'utiliser des catalyseurs nickel-oxyde de nickel ou nickel sur silice/alumine ou rhodium sur carbone ne correspond pas à un choix arbitraire mais correspond à une sélection nécessaire pour obtenir l'effet technique recherché.

Exemples comparatifs 3 et 4 :

Des essais ont été réalisés avec des catalyseurs à base de ruthénium et d'iridium supportés sur carbone (Ru/C et Ir/C) dans les mêmes conditions opératoires que celles décrites à l'exemple 1. La décomposition de l'hydrazine et de l'hydrazine substituée est extrêmement lente, de l'ordre de plusieurs jours, et on n'observe aucune sélectivité.

Exemple comparatif 5 :

Le même essai a été effectué avec du Nickel de Raney (48 % en poids de nickel et 52 % en poids d'aluminium). La décomposition de l'hydrazine et de l'hydrazine substituée est rapide (de l'ordre de 30 minutes) mais aucune sélectivité n'est observée.

## Revendications

1. Procédé de décomposition sélective de l'hydrazine dans un mélange hydrazine/hydrazine substituée/eau, **caractérisé en ce que** l'on introduit dans le mélange un catalyseur choisi dans le groupe constitué par le nickel supporté sur silice/alumine, le mélange nickel-oxyde de nickel supporté sur silice/alumine et le rhodium supporté sur carbone, puis **en ce que** l'on chauffe le mélange à une température comprise entre 60°C et 120°C.

2. Procédé selon la revendication 1, **caractérisé en ce que** le catalyseur contient de 0,5 % à 70 % en poids d'élément métal par rapport au poids total du catalyseur.

3. Procédé selon la revendication 2, **caractérisé en ce que** le catalyseur contient de 45 % à 70 % en poids d'élément métal par rapport au poids total du catalyseur.

4. Procédé selon la revendication 1, **caractérisé en ce que** l'hydrazine substituée est une hydrazine de formule (I)

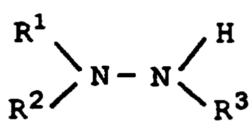
(I)

dans laquelle :

- $R^1$, $R^3$ représentent un atome d'hydrogène ou un groupe alkyl en $C_1$ - $C_6$, linéaire ou ramifié, substitué ou non,
- $R^2$ représente un groupe alkyl en $C_1$ - $C_6$, linéaire ou ramifié, substitué ou non,
- $R^1$ et $R^2$ pouvant former ensemble, avec l'atome d'azote auquel ils sont liés, un hétérocycle, et,
- $R^2$ et $R^3$, lorsque $R^1$ représente un atome d'hydrogène, pouvant former ensemble, avec les deux atomes d'azote, un hétérocycle.

5. Procédé selon la revendication 4, **caractérisé en ce que** $R^1$ et $R^3$ représentent un atome d'hydrogène et $R^2$ représente le groupe méthyl.

6. Procédé selon la revendication 1, **caractérisé en ce que** le catalyseur est le mélange nickel-oxyde de nickel supporté sur silice/alumine.

7. Procédé selon la revendication 1, **caractérisé en ce que** l'on chauffe le mélange réactionnel à une température comprise entre 80°C et 90°C.

8. Procédé selon la revendication 1, **caractérisé en ce que** la quantité d'hydrazine est comprise entre 0,01 mole et 1 mole par mole d'hydrazine substituée.

9. Procédé selon la revendication 1, **caractérisé en ce que** la teneur en eau du mélange hydrazine/hydrazine substituée/eau est comprise entre 10 % et 90 % en poids par rapport au poids total du mélange.

## Patentansprüche

1. Verfahren zur selektiven Zersetzung von Hydrazin in einer Mischung aus Hydrazin/substituiertem Hydrazin/Wasser, **dadurch gekennzeichnet, dass** man in die Mischung einen Katalysator einführt, der ausgewählt ist aus der Gruppe bestehend aus Nikkel, getragen auf Siliciumdioxid/Aluminiumoxid, der Mischung Nickel/Nickeloxid, getragen auf Siliciumdioxid/Aluminiumoxid, und Rhodium, getragen auf Kohlenstoff, dann **dadurch**, dass man die Mischung bei einer Temperatur zwischen 60°C und 120°C einschließlich erwärmt.

2. Verfahren nach Anspruch 1, **dadurch gekennzeichnet, dass** der Katalysator 0,5 bis 70 Gew.-% Metallelement, bezogen auf das Gesamtgewicht des Katalysators, enthält.

3. Verfahren nach Anspruch 2, **dadurch gekennzeichnet, dass** der Katalysator 45 bis 70 Gew.-% Metallelement, bezogen auf das Gesamtgewicht des Katalysators, enthält.

4. Verfahren nach Anspruch 1, **dadurch gekennzeichnet, dass** das substituierte Hydrazin ein Hydrazin der Formel (I) ist

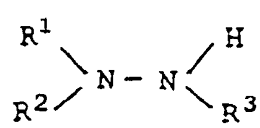
(I)

in der:

$R^1$, $R^3$ ein Wasserstoffatom oder eine lineare oder verzweigte, substituierte oder unsubstitu-

ierte ($C_1$-$C_6$)-Alkylgruppe darstellen,
$R^2$ eine lineare oder verzweigte, substituierte oder unsubstituierte ($C_1$-$C_6$)-Alkylgruppe darstellt,
$R^1$ und $R^2$ zusammen mit dem Stickstoffatom, an das sie gebunden sind, einen Heterocyclus bilden können und
$R^2$ und $R^3$ , wenn $R^1$ ein Wasserstoffatom darstellt, zusammen mit den zwei Stickstoffatomen einen Heterocyclus bilden können.

5. Verfahren nach Anspruch 4, **dadurch gekennzeichnet, dass** $R^1$ und $R^3$ ein Wasserstoffatom darstellen und $R^2$ eine Methylgruppe darstellt.

6. Verfahren nach Anspruch 1, **dadurch gekennzeichnet, dass** der Katalysator die Mischung Nikkel/Nickeloxid, getragen auf Siliciumdioxid/Aluminiumoxid, ist.

7. Verfahren nach Anspruch 1, **dadurch gekennzeichnet, dass** man die Reaktionsmischung bei einer Temperatur zwischen 80°C und 90°C einschließlich erwärmt.

8. Verfahren nach Anspruch 1, **dadurch gekennzeichnet, dass** die Hydrazinmenge zwischen 0,01 Mol und 1 Mol einschließlich pro Mol substituiertes Hydrazin beträgt.

9. Verfahren nach Anspruch 1, **dadurch gekennzeichnet, dass** der Wassergehalt der Mischung aus Hydrazin/substituiertem Hydrazin/Wasser zwischen 10 und 90 Gew.-% einschließlich, bezogen auf das Gesamtgewicht der Mischung, beträgt.

## Claims

1. Process for the selective decomposition of hydrazine in a hydrazine/substituted hydrazine/water mixture, **characterized in that** a catalyst chosen from the group consisting of nickel supported on silica/alumina, the nickel-nickel oxide mixture supported on silica/alumina and rhodium supported on carbon is introduced into the mixture and then **in that** the mixture is heated at a temperature of between 60°C and 120°C.

2. Process according to Claim 1, **characterized in that** the catalyst comprises from 0.5% to 70% by weight of metal element with respect to the total weight of the catalyst.

3. Process according to Claim 2, **characterized in that** the catalyst comprises from 45% to 70% by weight of metal element with respect to the total weight of the catalyst.

4. Process according to Claim 1, **characterized in that** the substituted hydrazine is a hydrazine of formula (I)

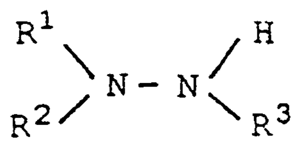

(I)

in which:

- $R^1$ and $R^3$ represent a hydrogen atom or a substituted or unsubstituted and linear or branched $C_1$-$C_6$ alkyl group,
- $R^2$ represents a substituted or unsubstituted and linear or branched $C_1$-$C_6$ alkyl group,
- it being possible for $R^1$ and $R^2$ together to form, with the nitrogen atom to which they are bonded, a heterocycle, and
- it being possible for $R^2$ and $R^3$, when $R^1$ represents a hydrogen atom, together to form, with the two nitrogen atoms, a heterocycle.

5. Process according to Claim 4, **characterized in that** $R^1$ and $R^3$ represent a hydrogen atom and $R^2$ represents the methyl group.

6. Process according to Claim 1, **characterized in that** the catalyst is the nickel-nickel oxide mixture supported on silica/alumina.

7. Process according to Claim 1, **characterized in that** the reaction mixture is heated at a temperature of between 80°C and 90°C.

8. Process according to Claim 1, **characterized in that** the amount of hydrazine is between 0.01 mol and 1 mol per mole of substituted hydrazine.

9. Process according to Claim 1, **characterized in that** the content of water in the hydrazine/substituted hydrazine/water mixture is between 10% and 90% by weight with respect to the total weight of the mixture.